# EUROPEAN PATENT APPLICATION

(11) **EP 1 447 410 A1**
(43) Date of publication of application: **18.08.2004**
(21) Application number: 03075366.9
(22) Date of filing: 06.02.2003
(51) Int. Cl.: C07K 14/415, C12N 15/29, C12N 15/82

(54) **Control of plant growth and developmental processes**

(71) Applicant: Universiteit Leiden, 2312 AV Leiden (NL); Stichting Binair Vector Systeem, 2343 RS Oegstgeest (NL)
(72) Inventor: Hooykaas, Paul Jan Jacob, 2343 RS Oegstgeest (NL); van Attikum, Haico, 2334 EV Leiden (NL); Bundcock, Paul, 1106 HJ Amsterdam (NL)
(74) Representative: Prins, Adrianus Willem, Mr. Ir.

(57) **Abstract**

The invention a method to modulate plant growth or developmental processes in a plant or plant cell, more in particular organogenesis and/or embryogenesis, through the provision of a plant protein which can act a transcriptional regulator of said processes. The invention further provides a plant and propagating material thereof which contains in its genome nucleotide sequence encoding a protein of the invention, said protein characterised in that it comprises the amino acid sequence PRGRPXGSKNK, wherein X is A, P, L or R.

## Description

The invention relates to the field of plant biotechnology. In particular it relates to the area of controlling plant growth and developmental processes, such as organogenesis and/or embryogenesis, more specifically through the expression of genes encoding transcriptional regulators central to these processes.

### Introduction

Morphogenesis of plants *in vitro* is a complex developmental process which involves differential gene expression, cell proliferation, and differentiation. Given the right conditions, plant cells undergo morphogenic development leading to plant regeneration, such as somatic embryogenesis and organogenesis. Plant morphogenesis requires tight co-ordination among a range of processes. Because plant cells do not migrate, morphogenesis is ultimately dependent on appropriate directional cell expansion and division. Expansion and division (of cells) must be integrated with positional information such that new structures form at particular locations and in the appropriate orientation. In addition growth must be co-ordinated among large numbers of cells that are not directly in contact with one another to produce bilaterally symmetrical organs such as leaves, sepals, petals and ovules.

Embryogenesis, in plants as in animals, is an exquisitely controlled sequence of events, wherein subtle biochemical alterations mediate major changes in form and function. In plants the process of embryo formation in the seed can be mimicked in the laboratory in a technique called somatic embryogenesis. Somatic embryogenesis is the most direct way to regenerate plants from single cells or protoplasts. Somatic embryogenesis has been defined as a non-sexual developmental process that produces a bipolar embryo from somatic tissue. Developmental stages similar to zygotic embryogenesis occur and yield an embryo with no vascular connections to the parental tissue. Different patterns in the origins of somatic embryos have been distinguished from *in vitro* grown explants. These include direct production of somatic embryos from the explant cells called pre-embryonically determined cells, and indirect production of somatic embryos from induced embryogenically determined cells in unorganised callus/tissue mass.

By using cell suspensions, several genes expressed specifically during somatic embryogenesis have been isolated. Mutational analysis in *Arabidopsis* and maize has helped to identify genetic loci that control embryo maturation and germination. The largest class of loci is that encoding proteins regulating hormone production or sensitivity. Mutation of loci encoding proteins involved in ABA synthesis and perception disrupt embryo maturation, desiccation tolerance and embryo dormancy. In contrast, mutations that decrease levels of active gibberellic acid (GA) influence germination potential. Several other loci have also been identified that specifically affect seed maturation and germination but do not appear to be directly related to hormone synthesis or signalling.

A typical angiosperm seed initially consists of three major components, the embryo, the endosperm and the maternal seed coat. Seed development begins with a double fertilization event, in which one sperm cell nucleus fuses with the egg cell nucleus to form the embryo, and a second sperm cell nucleus fuses with two central cell nuclei to form the endosperm. Embryo development itself can be separated into three developmental phases. The first phase of embryo development is one of cell division and morphogenesis, which serves to establish the major tissue types and organ systems of the mature plant. The second phase encompasses a period of rapid cell expansion and is characterized by the synthesis of storage reserves that sustain the embryo during germination and early seedling development. In the final phase of embryo development, the embryo becomes desiccated and enters into a period of developmental arrest or dormancy. All of the above events normally take place while the seed remains attached to the maternal plant.

Although, as a rule, higher plants routinely reproduce sexually, i.e., by way of gametic fusion, there are, among certain types of plants, episodes of various types of asexual reproduction. Some varieties may typically be reproduced asexually by vegetative propagation. This phenomenon is frequently used by plant breeders in plants with poor seed set; it may also be used to eliminate an undesired genetic variability which may result from seed propagation. Vegetative propagation may be achieved by roots, tubers, stolons, rhizomes, stem or leaf cuttings, or tissue culture; those plants obtained in this manner are, absent a somatic mutation, genotypically and phenotypically identical to the parent plant. A number of well-known commercial crops are routinely produced in this manner. For example, stem sections are frequently used in the propagation of sugarcane, which only rarely produces flowers in non-tropical regions. Similarly, roots and tubers are employed in the production of root crops such as cassava, sweet potatoes, potatoes, and yams.

Many plant species are capable of producing embryos in the absence of fertilization. This process of asexual embryo development may occur naturally, for example on the leaf margins of Bryophyllum (Yarborough, 1923) and Malaxis (Taylor, 1967), or within the ovule of apomictic plants (Koltunow, 1995). Apomixis refers to the production of a seed from the maternal ovule tissues in the absence of egg cell fertilization. Asexual embryo development may also be induced in vitro from gametophytic or somatic tissue (Mordhorst et al., 1997) or may be induced by genetic modification of gene expression (Ogas et al., 1997; Lotan et al., 1998).

Three major mechanisms of apomixis, diplospory, apospory and adventitious embryony, have been observed. Each mechanism differs with respect to the source of the cell that gives rise to the embryo and with respect to the time during ovule development at which the apomictic process is initiated. Diplospory and apospory are considered gametophytic forms of apomixis as they involve the formation of diploid embryo sacs. Adventitious embryony does not involve the production of a mitotically-derived embryo sac.

In diplospory, the megaspore mother cell does not undergo normal meiosis, but rather divides mitotically to produce a diploid embryo sac instead of the normal haploid embryo sac. One of the cells of the embryo sac functions as the egg cell and divides parthenogenetically (without fertilization) to form an embryo. In some species the unreduced polar nuclei of the embryo sac may fuse to form the endosperm (autonomous endosperm production), the nutritive tissue of the seed, while in other species pollination is necessary for endosperm production (pseudogamy).

In aposporous apomicts, parthenogenic embryos are produced from additional cells, the aposporous initials, that differentiate from the nucellus. As with the megagametophyte of diplosporous species, the aposporous initial undergoes mitotic divisions to produce a diploid embryo sac. Aposporous embryos are not derived from the megagametophyte and can therefore co-exist within a single ovule with sexually-derived embryos. Autonomous production of endosperm is rare in aposporous species. Aposporous apomicts therefore depend on fertilization of the polar nuclei of a meiotically-derived embryo sac for the production of endosperm.

With adventitious embryony, embryos are formed directly from sporophytic ovule tissue, such as the integuments or nucellus, via parthenogenesis. Seeds derived from species exhibiting adventitious embryony generally contain multiple asexually-derived embryos and may also contain a single sexually-derived embryo. Plants exhibiting adventitious embryo also rely on the presence of a meiotically-derived embryo sac within the same ovule for endosperm formation.

In most plant species, the apomictic trait appears to be under the control of a single dominant locus. This locus may encode one or more developmental regulators, such as transcription factors, that in sexually reproducing plants function to initiate gene expression cascades leading to embryo sac formation and/or embryogenesis, but which are heterochronically or ectopically expressed in apomictic plants (Peacock, 1992; Koltunow, 1993; Koltunow et al, 1995).

Apomixis is a valuable trait for crop improvement since apomictic seeds give rise to clonal offspring and can therefore be used to genetically fix hybrid lines. The production of hybrid seed is a labour intensive and costly procedure as it involves maintaining pop ulations of genetically pure parental lines, the use of separate pollen donor and male-sterile lines, and line isolation. Production of seed through apomixis avoids these problems in that once a hybrid has been produced, it can be maintained clonally, thereby eliminating the need to maintain and cross separate parental lines. The use of apomictic seed also provides a more cost effective method of multiplying vegetatively-propagated crops, as it eliminates the use of cuttings or tissue culture techniques to propagate lines, reduces the spread of diseases which are easily transmitted through vegetatively-propagated tissues, and in many species reduces the size of the propagule leading to lower shipping and planting costs.

Although apomixis occurs in a wide range of plant species, few crop species are apomictic. Attempts to introduce apomictic traits into crop species by introgression from wild relatives (Ozias-Akins, et al., 1993; WO 97/10704; WO 97/11167) or through crosses between related, but developmentally divergent sexual species (WO 98/33374), have not yielded marketable products. Other approaches have focused on the identification of gene sequences that may be used to identify or manipulate apomictic processes (WO 97/43427; WO 98/36090), however these approaches have not led to methods for the routine production of apomictic plants.

Asexually-derived embryos similar to those formed 'in vivo' by apomixis, can be induced to form in culture from many gametophytic and somatic plant tissues (Yeung, 1995). Somatic embryos can be obtained from culture of somatic tissues by treating them with plant growth regulators, such as auxins, or auxins in combination with cytokinins. Embryos can also be induced to form in culture from the gametophytic tissues of the ovule (gynogenesis) and the anther (androgenesis, pollen or microspore embryogenesis), either by the addition of plant growth regulators or by a simple stress treatment.

Several mutants have been identified that may be used to induce efficient production of embryos in vitro. These include recessive arabidopsis mutants with altered shoot meristems, for example primordia timing (pt), clavata (clv)1 and clv3, which were shown to enhance embryogenic callus formation when seedlings were germinated in the presence of auxin (Mordhorst et al., 1998). The altered expression of two arabidopsis genes, LEAFY COTYLEDON (LEC1; WO 98/37184, Lotan et al., 1998) and PICKLE, have been shown to promote the production of somatic embryos in the absence of added growth regulators. The LEC1 gene encodes a homologue of the HAP3 subunit of a CCAAT box-binding transcription factor (CBF). The LEC1 gene controls many aspects of zygotic embryo development including desiccation tolerance and cotyledon identity. Ectopic over-expression of the LEC1 gene in a lec1 mutant background can result in the production of transgenic lines that occasionally form embryo-like structures on leaves.

These embryo-like structures express genes, such as those encoding seed storage proteins and oil body proteins, which are normally preferentially expressed in developing embryos. Plants containing a recessive mutant PICKLE gene produce a thickened, primary root meristem. Mutant pickle roots produce embryo-forming callus when the root tissue is separated from the rest of the plant and placed on minimal medium without growth regulators (Ogas et al., 1997). Mutant pickle roots show morphological characteristics of developing seeds, such as oil bodies and, as with LEC1 over-expressers, accumulate genes preferentially expressed in developing seeds.

Efficient production of apomictic seed is only likely to be realised through the identification and subsequent modification of developmental regulators, such as transcription factors, that are known to activate gene expression cascades leading to embryogenesis in both sexually-reproducing and apomictic plants. The present invention addresses this need.

Plant micropropagation is an efficient method of propagating disease-free, genetically uniform and massive amounts of plants *in vitro*. Micropropagation can be achieved by direct organogenesis from roots or shoots or via more complicated procedures involving regeneration of plant organs from less organized somatic tissue. When spontaneous vegetative propagation does not occur in a species, micropropagation requires specific plant growth regulators and environmental conditions. Furthermore the choice of genotype and explant is crucial in obtaining regenerative response. However, somatic embryogenesis as outlined above has several advantages over micropropagation via (ectopic) organogenesis as a practical means of propagation.
It bypasses the necessity of timely and costly manipulations of individual explants to obtain organogenesis. It does not require the time consuming subculturing steps to increase clonal stocks and may overcome difficulties with micropropogation of plant species because of problems with rooting of explants. Somatic embryogenesis may accelerate the introduction of improved clones into commercial production as somatic embryos can be encapsulated and handled as seeds. Somatic embryos or embryogenic cultures are thus appropriate materials for cryopreservation or for the development of artificial seeds, in commercial plant production.

However, somatic embryo quality is still the primary barrier to the operational use of somatic embryos as artificial seeds for most species. Although large quantities of somatic embryos can be rapidly produced, normal plants are difficult to obtain from these embryos due to asynchronous maturation. Often, somatic embryos discontinue growth prematurely, resulting in embryos which do not germinate or germinate slowly. Additionally the phenotype of the regenerated plants may be altered. As a result, in many cases regeneration *via* organogenesis is the only option.

While histology of plant growth and development has been well characterised, little is known about how these processes are regulated at the molecular level. To date, a broad based and unbiased view of gene expression during embryogenesis and organogenesis in animals and plants is still lacking. As mentioned previously, plant growth and developmental processes such as somatic embryogenesis and organogenesis are fraught with problems. The unpredictability of these regeneration processes and the difficulty associated with regenerating plants from callus, cell suspension cultures and root and shoot explants, for many species (e.g. grape, conifer species etc.), is still a major problem. The capability to regulate gene expression controlling essential developmental processes, like embryogenesis or organogenesis, through the use of transcriptional regulators would circumvent many of the problems addressed above. The ability to control at the molecular level critical steps in the rejuvenation of explant source materials is crucial for the effective use of somatic embryogenesis and organogenesis in commercial plant improvement programs.

### Summary of the invention

The invention provides a plant protein involved in transcriptional regulation characterised in that it comprises the amino acid sequence PRGRP**X**GSKNK, wherein **X** is A, P, L or R. Furthermore the invention provides a plant protein characterised in that in addition it also comprises a DUF296 domain. A specific embodiment of the present invention is a plant protein as mentioned above, characterised in that it has at least 200 amino acids, whereby the amino acid sequence PRGRPXGSKNK is present in the N-terminal part, and wherein the DUF296 domain is separated from said sequence by about 13-20 amino acid residues. In a specific aspect of the invention said plant protein according to the invention is characterised in that it comprises the amino acid sequence [NP_191115.1{number from Genbank}] or [NP_177776.1], or a functional part thereof.

Also provided in the invention is a nucleotide sequence encoding a protein as mentioned above. In another specific aspect the invention provides a nucleotide sequence, characterised in that said nucleotide sequence comprises the nucleotide sequence [NM_115413.1] or [NM_106300.1]. Additionally the invention provides a vector comprising a nucleotide sequence of the invention, a transformed plant cell or plant comprising a vector according to the invention and seed obtained from a transformed plant comprising a vector according to the invention.

The invention further provides a method to modulate plant growth or developmental processes in a plant or plant cell, characterised in that a protein or nucleotide sequence or a vector according to the invention is introduced into said plant or plant cell. Preferred is that said developmental process comprises organogenesis and/or embryogenesis, preferably ectopic organogenesis or somatic embryogenesis. In another aspect is provided a method according to the invention wherein said plant or plant cell is provided with a growth regulator, preferably a brassinosteroid.

The invention now provides a method for producing asexually derived embryos comprising: i) transforming a plant cell with the vector according to the invention; ii) growing said plant cell to produce tissue; iii) selecting said tissue for the presence of a nucleotide sequence of the invention; and iv) assaying said tissue for asexual embryo production; v) maintaining or growing isolated mature or immature embryos in culture.

Also included in the invention is a method of producing asexually derived embryos comprising: i) introducing into said plant cell a protein according to the invention to produce a modified plant cell; ii) growing said modified plant cell to produce tissue; and iii) assaying said tissue for asexual embryo formation; v) maintaining or growing isolated mature or immature embryos in culture.

A further embodiment of the invention is a method for producing an apomictic plant comprising: i) transforming a plant with the vector according to the invention to produce a transformed plant; or ii) introducing into said plant a protein according to the invention; ii) selecting said transformed plant for the presence of a nucleotide sequence of the invention; and/or iii) assaying said transformed plant for asexual embryo production; iv) maintaining or growing isolated mature or immature embryos in culture; v) production of an apomictic plants from the mature embryos of step iv.

In particular said invention relates to an above method wherein said step of assaying involves assaying for asexually derived embryos, somatic embryos, gametophytically derived embryos, adventitious embryony, diplospory or for haploid parthenogenesis of the embryo sac.

The invention further provides a method for enhancing the regenerative capacity of a plant comprising: i) transforming a plant cell with the vector according to the invention, or introducing into said plant cell a protein according to the invention; to produce a modified plant cell: ii) growing said modified plant cell to produce tissue; and iii) assaying said tissue for enhanced regeneration as compared to wild-type tissue; iv) regeneration of complete plants from said tissue.

Preferably the step of growing said transformed plant cell, the step of assaying said tissue, or both the step of growing said transformed plant cell and the step of assaying said tissue are carried out in the absence of an additional growth regulator.

In yet another aspect the invention provides a method of selecting a modified plant comprising; i) transforming a normally non-regenerative plant with a vector according to the invention; or introducing into said plant a protein according to the invention, to produce said modified plant; and ii) determining whether said modified plant is able to regenerate under conditions in which said normally non-regenerative plant does not regenerate.

The invention further provides a method of producing a protein of interest comprising; i) transforming a plant with a vector according to the invention and a second vector comprising a nucleotide sequence encoding a protein of interest under the control of a regulatory element, wherein said regulatory element is induced by the expression product of a nucleotide sequence according to the invention; to produce a transformed plant; ii) selecting said transformed plant for occurrence of a nucleotide sequence according to the invention; and iv) growing said transformed plant in order to produce said protein of interest.

Preferably said protein of interest is selected from the group consisting of a pharmaceutically active protein, antibody, industrial enzyme, protein supplement, nutraceutical, storage protein, an enzyme involved in oil biosynthesis, animal feed, and animal feed supplement.

The invention also concerns a method to modulate plant growth or developmental processes in a plant or plant cell, characterised in that a nucleotide sequence of the invention or a vector comprising a nucleotide sequence of the invention is introduced into said plant or plant cell. In a preferred embodiment said developmental process comprises organogenesis and/or embryogenesis, preferably ectopic organogenesis or somatic embryogenesis.

Also provided in the invention is a method to modulate plant growth or developmental processes in a plant or plant cell as mentioned above, further providing said plant or plant cell with a growth regulator, preferably a brassinosteroid. A further aspect of the invention is the use of a nucleotide sequence according to the invention or a vector according comprising a nucleotide sequence of the invention, to modulate plant growth or developmental processes in a plant or plant cell. In a preferred embodiment said developmental process comprises organogenesis and/or embryogenesis, preferably ectopic organogenesis or somatic embryogenesis. Further provided is the use of a nucleotide sequence or a vector according to the invention, comprising a nucleotide sequence of the invention, in combination with a growth regulator, preferably a brassinosteroid, to modulate plant growth or developmental processes in a plant or plant cell.

A specific embodiment of the invention is a transgenic plant or plant cell obtainable by a method according to the invention. Further provided is the use of material from a transgenic plant or plant cell according to the invention, to modulate plant growth or developmental processes, preferably ectopic organogenesis or somatic embryogenesis, in a non-transformed plant or plant cell.

### Description of figures

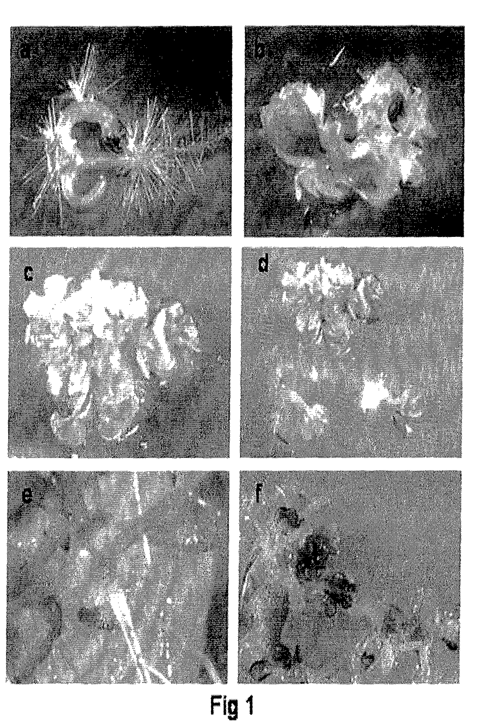

Figure 1: A gene construct with the cDNA corresponding to NM_115413.1 under control of the CaMV 35S promoter can lead to ectopic organogenesis and formation of somatic embryos. Growth of plant material is on ½ MS medium without hormones, supplemented with 1% sucrose. The material shown is from homozygous lines.
a: ectopic root growth on cotyledons (13 days after germination (dag)).
b: seedling with embryonic structures (18 dag).
c-d: seedling which has developed into a cluster of somatic embryos (c: detail of the cluster at 28 dag; d: two embryos are shown that were taken from the cluster several days earlier and which now show the outgrowth of roots).
e-f: seedlings (28 dag) showing aberrant cotyledons with various stages of somatic embryo development. Even in homozygous lines a variety of phenotypes was observed (see f.).

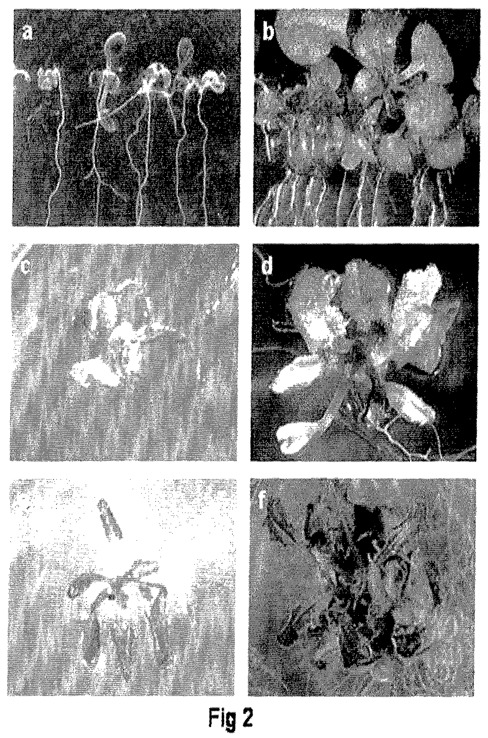

Figure 2: A gene construct with the cDNA corresponding to NM_106300.1 under control of the CaMV 35S promoter can lead to severe developmental effects, most notably deformation of cotyledons. Growth of plant material is on ½ MS medium without hormones, supplemented with 1% sucrose. The material shown is from homozygous lines, except for the primary transformants shown in panel e.
a: variation of phenotypes, ranging from nearly wild-type growth to severe growth retardation (14 dag).
b: variation of phenotypes at 28 dag.
c-d: different seedlings with callus formation on cotyledons and older true leaves (seedling in c. at 28 dag, in d. at 33 dag)
e: seedling with aberrant cotyledon and leaf development (28 dag)
f: primary transformant with leaf deformation and delayed inflorescence development.

Figure 3: After germination of wild-type arabiodopsis seeds (ecotype Columbia) on ½MS medium containing 24-epibrassinolide (at 4 µM), growth aberrations resembling those seen in NM_115413.1 or NM_106300.1 overexpressing seedlings can be observed.
a and c: controls (0.1% DMSO) at 14 and 28 dag.
b and d: seedlings on 4 µM 24-epibrassinolide.

Figure 4: *In vitro* culture of seedlings in liquid MS medium (MS-4) containing 4.4 µM 2,4-dichlorophenoxyacetic-acid (2,4-D) as described by Mordhorst et al. (Genetics 149, 549-563 (1998)).
Top panel: seedlings of a homozygous control line containing an empty vector (i.e. without a cDNA sequence downstream of the CaMV 35S promoter) at 17 dag (phenotype similar to untransformed Columbia, which is not shown here).
Bottom panel: seedlings of a line homozygous for a construct expressing NM_115413.1 under control of the CaMV 35S promoter. Note the large meristematic apical zone in these seedlings and their narrow cotyledons compared to the formation of unorganized callus on the cotyledons of the control line (top panel)

### Detailed description of the invention

In the present invention it is shown that plant proteins comprising a single sequence motif/domain - PRGRPXGSKNK (wherein X is A, P, L or R) act as transcriptional regulators playing a central role in plant growth and developmental processes. In the dicotelydenous plant species *Arabidopsis thaliana* 15 novel genes encoding plant proteins as mentioned above can be found (8 with A, 6 with P, and 1 with R substitutions), ranging in size from 206-339 amino acids. These sequences have the following accession numbers from Genbank [nucleotide accession number NM_115413.1, NM_117526.1, NM_111328.1, NM_106300.1, NM_130105.1, NM_101943.1, NM_117890.2, NM_119705.1, NM_117275.1, NM_118410.1, NM_115951.1, NM_129855.2, NM_129079.1, NM_124348.1 and NM_101316.1] coding for [protein accession numberNP_191115.1, NP_567432.1, NP_566232.1, NP_177776.1, NP_182067.1, NP_173514.1, NP_193515.1, NP_195265.1, NP_192942.1, NP_194012.1, NP_191646.1, NP_181822.1, NP_181070.1, NP_199781.1 and NP_172901.1, respectively]. Further in the monocotelydenous species *Oryza sativa* (rice) 11 novel genes encoding said plant proteins have been identified (6 with P, 2 with A and 3 with L substitutions) by t-blast searches in the Gramene database (www.gramene.org), as listed [AAAA01003101.1, AAAA01003524.1, AAAA01017331.1, AAAA01000383.1, AAAA01000486.1, AAAA01009427.1, AAAA01003389.1, AAAA01000935.1, AAAA01000192.1, AAAA01003191.1 and AAAA01000868.1]. The sequence motif PRGRPXGSKNK (wherein X is A, P, L or R) is found in the N-terminal region of the protein.

Some of the transcriptional regulator genes identified comprising the sequence motif/domain PRGRPXGSKNK (wherein X is A, P, L or R), also comprise a single DUF296 domain (http://www.sanger.ac.uk/cgi-bin/Pfam/getacc?PF03479). This DUF296 domain is located approximately 13-20 amino acid residues downstream of the last lysine residue of the PRGRPXGSKNK (wherein X is A, P, L or R) motif. This DUF296 domain comprises at least 120 amino acid residues. Until the present invention no clear function had been assigned to any of the above mentioned proteins, or domains. Since the DUF296 domain is found in proteins that contain one or more PRGRPXGSKNK motifs, a DNA-binding function for these proteins as a whole can be expected (http://pfam.wustl.edu)

In some of these proteins it was observed that the glycine (G) residue prior to the cysteine (C) residue conserved in most eukaroyte DUF296 signature sequences was deleted giving rise to a novel plant DUF296 signature sequence - "**D** (16x) **C**", where **C** is the conserved cystine residue, and x corresponds to any amino acid residue (e.g. **D**ivekimaFarqrprgi**Cv**lSaiG). It was observed that in some cases the conserved cysteine residue of the above mentioned signature sequence was replaced by a serine residue (e.g. "**D**(16 x) **S**).

As disclosed herein, proteins comprising the features as mentioned above, that is a single motif/domain PRGRPXGSKNK (wherein X is A, P, L or R) alone or comprising both the motif/domain PRGRPXGSKNK (wherein X is A, P, L or R) in combination with the DUF296 domain, are believed to be involved in transcriptional regulation of growth and developmental processes in plants, more specifically ectopic organogenesis or somatic embryogenesis. More in particular, proteins of the invention, comprising the motif/domain PRGRPXGSKNK (wherein X is A, P, L or R) in combination with the DUF296 domain, where the conserved cysteine residing in the DUF296 domain was substituted for a serine residue (e.g. "D (x16) S "), when expressed under the control of an effective plant promoter, were found to alter plant morphology. One of them could even incite ectopic embryo development.

In a specific aspect of the invention, a plant protein according to the invention is characterised in that it comprises the amino acid sequence [NP_191115.1] or [NP_177776.1] or a functional part thereof. A functional part of said protein is defined as a part of said protein which has the same biological activity in kind, but not necessarily in amount. The word protein means a sequence of amino acids connected through peptide bonds. Polypeptides or peptides are also considered to be proteins.

Variants of proteins of the invention are proteins obtained from the proteins as mentioned above, by replacing adding/deleting one or more amino acids, while still retaining biological activity. Deliberate amino acid substitution may be made on the basis of similarity in polarity, charge, solubility, hydrophobicity, and/or the amphipathetic nature of the residues as long as the biological activity of the polypeptide is retained. As long as the changes to the amino acid sequence of the protein do not altogether abolish the activity of the protein, such variants are embraced in the present invention.

It is clear to a person skilled in the art that through the provision of accession numbers for rice and *Arabidopsis* proteins of the invention as disclosed, which give the person skilled in the art access to the amino acid sequence and the nucleotide sequence coding for these proteins, that homologous proteins derived from other plant sources (also called orthologues, paralogues) can be easily found. Thus also encompassed by the present invention are homologous proteins of the proteins of the invention derived from other plant sources. Orthologues or paralogues of proteins of the invention can be identified through searching established public databases such as the NCBI databases [http://www.ncbi.nlm.nih.gov/].

Further part of the invention are polynucleotides coding for [NP_191115.1] and [NP_177776.1] and polynucleotides, encoding for proteins which have a biological function identical to the function of a protein of the invention, which are the product of amplification from a nucleotide library using primer pairs which selectively hybridise under stringent conditions to loci within the above mentioned nucleotide sequences. The primer length in nucleotides is selected from the group of integers consisting of from at least 15 to 50. Those of skill in the art will recognise that a lengthened primer sequence can be employed to increase specificity of binding (i.e. annealing) to a target sequence. Stringent conditions in this respect means a reaction at a temperature of between 60°C and 65°C in 0.3 strength citrate buffered saline containing 0.1% SDS followed by rinsing at the same temperature with 0.3 strength citrate buffered saline containing 0.1% SDS.

Thus, also part of the invention are polynucleotides which selectively hybridise, under selective hybridisation conditions, to one or more of the above discussed nucleotide sequences, and which code for an amino acid sequence which has a biological function similar to the function of a protein according to the invention. Another way to indicate hybridisation potential is on sequence identity. In this sense, the present invention provides also for nucleotide sequences which have a percentage of identity related to the above mentioned sequences of 40% to 95% . Thus, for example, the percentage of identity can be at least, 40%, 45%, 50%, 55%, 60%, 65%, 70%, 75%, 80%, 85%, 90%, or 95% . Sequence identity on nucleotide sequences can be calculated by using the BLASTN computer program (which is publicly available, for instance through the National Center for Biotechnological Information, accessible via the internet on http://www.ncbi.nlm.nih.gov/) using the default settings of 11 for wordlength (W), 10 for expectation (E), 5 as reward score for a pair of matching residues (M), -4 as penalty score for mismatches (N) and a cutoff of 100.

In a preferred embodiment of the invention said nucleotide sequence encoding a protein of the invention comprises the nucleotide sequence coding forNP_191115.1 or coding forNP_177776.1. Also part of the invention are nucleotide sequences which are conservatively modified variants of the nucleotide sequences of the invention (denoted by accession numbers), or polymorphic variants thereof. It may be advantageous to produce nucleotide sequences according to the invention, or derivatives thereof possessing a substantially different codon usage. It is known by those skilled in the art that as a result of degeneracy of the genetic code, a multitude of gene sequences, some bearing minimal homology to the nucleotide sequences of any known and any naturally occurring genes may be produced. The invention contemplates each and every possible variation including "silent variations" of the nucleotide sequences that could be made by selecting combinations based on possible codon choices. These combinations are made in accordance with the standard triplet genetic code as applied to the nucleotide sequence of naturally occurring gene sequences, and all such variations are to be considered as being specifically disclosed. Thus included in the invention are altered nucleotide sequences of the invention, arising from deletions, insertions, substitutions of different nucleotides resulting in polynucleotides that encode the same or are functionally equivalent. Further the nucleotide sequences of the invention can be used, for example to selectively hybridise and detect allelic variants of the nucleotide sequences of the present invention. Additionally the present invention provides isolated nucleotide sequences comprising one or more polymorphic (allelic) variants of the nucleotide sequences of the invention.

Also provided in the invention are constructs having a nucleotide sequence of the invention under the control of a suitable promoter/regulatory element. By "regulatory element" it is meant those that include developmentally regulated, tissue specific, inducible and constitutive regulatory elements/promoters.

A regulatory element that is developmentally regulated, or controls the differential expression of a gene/nucleotide under its control, is activated within certain organs or tissues of an organ at specific times during the development of that organ or tissue. However, some regulatory elements that are developmentally regulated may preferentially be active within certain organs or tissues at specific developmental stages, they may also be active in a developmentally regulated manner, or at a basal level in other organs or tissues within the plant as well, such regulatory elements are considered "tissue specific". Regulatory elements may be found either upstream, within, downstream, or a combination thereof, of the coding region of a gene.

An inducible regulatory element is one that is capable of directly or indirectly activating transcription of one or more nucleotide sequences or genes in response to an inducer. In the absence of an inducer the nucleotide sequences or genes will not be transcribed. Typically the protein factor, that binds specifically to an inducible regulatory element to activate transcription, is present in an inactive form which is then directly or indirectly converted to the active form by the inducer. The inducer can be a chemical agent such as a protein, metabolite, growth regulator, herbicide or phenolic compound or a physiological stress imposed directly by heat, cold, salt, or toxic elements or indirectly through the action of a pathogen or disease agent such as a virus. A plant cell containing an inducible regulatory element may be exposed to an inducer by externally applying the inducer to the cell or plant such as by spraying, watering, heating or similar methods.

A constitutive regulatory element directs the expression of a gene throughout the various parts of a plant and continuously throughout plant development. Examples of known constitutive regulatory elements include promoters associated with the CaMV 35S transcript. (Odell et al., 1985, Nature, 313: 810-812), the rice actin 1 (Zhang et al, 1991, Plant Cell, 3: 1155-1165) and triosephosphate isomerase 1 (Xu et al, 1994, Plant Physiol. 106: 459-467) genes, the maize ubiquitin 1 gene (Cornejo et al, 1993, Plant Mol. Biol. 29: 637-646), the Arabidopsis ubiquitin 1 and 6 genes (Holtorf et al, 1995, Plant Mol. Biol. 29: 637-646), and the tobacco translational initiation factor 4A gene (Mandel et al, 1995 Plant Mol. Biol. 29: 995-1004).

The recombinant gene constructs may be inserted into vectors, which may be commercially available, suitable for transforming into plants and suitable for the expression of the gene product in the transformed cells. Preferably used are binary vectors which are useful for plant transformations using *Agrobacterium.*

To aid in identification of transformed plant cells, the constructs of this invention may be further manipulated to include plant selectable markers. Useful selectable markers include enzymes which provide for resistance to an antibiotic such as gentamycin, hygromycin, kanamycin, and the like. Similarly, enzymes providing for production of a compound identifiable by colour change such as GUS (beta-glucuronidase), fluorescence or luminescence, such as luciferase or green fluorescent protein are useful.

The invention thus provides a vector comprising a nucleotide sequence of the invention. Methods which are well known to those skilled in the art can be used to construct expression vectors containing a nucleotide sequence of the invention, and appropriate transcriptional and translational controls. These methods include *in-vitro* recombinant techniques. Such techniques are described in Sambrook *et al*., 1989. Molecular cloning a laboratory manual, cold spring Harbour press, Plain view, NY and Ausubel FM *et al*., (1989) Current protocols in molecular biology, John Wiley and Sons, New York, NY.

For example in order to express a biologically active polypeptide, or functional equivalents or fragments thereof, the nucleotide sequence encoding the polypeptide, is inserted into the appropriate expression vector (i.e. a vector that contains the necessary elements for the transcription or translation of the inserted coding sequence). Specific initiation signals may also be required for efficient translation of the polypeptides of the invention. These signals include the ATG initiation codon and adjacent sequences. In cases where the polypeptides, their initiation codons and upstream sequences are inserted into the appropriate expression vector, no additional translational control systems including the ATG initiation codon must be provided. Furthermore, the initiation codon must be in the correct reading frame to ensure transcription of the entire insert. Exogenous transcriptional elements and initiation codons can be of various origins, both natural and synthetic. The efficiency of expression may be enhanced by the inclusion of enhancers appropriate to the cell system in use (Scharf D et al (1994) Results Prob Cell Differ 20: 125-62; Bittner *et al*. (1987) *Methods in Enzymol* 153: 516-544).

In developing the expression cassette, the various fragments comprising the regulatory regions and open reading frame may be subjected to different processing conditions, such as ligation, restriction enzyme digestion, resection, *in-vitro* mutagenesis, primer repair, use of linkers and adapters and the like. Thus, nucleotide transitions, transversions, insertions, deletions and the like, may be performed on the DNA which is employed in the regulatory regions and/or open reading frame. The expression cassette may be wholly or partially derived from natural sources endogenous to the host cell.

The nucleotide sequences of the present invention can be engineered in order to alter the coding sequence for a variety of reasons, including but not limited to, alterations which modify the cloning, processing, and/or expression of the gene product. For example mutations may be introduced using techniques which are well known in the state of the art, e.g. site directed mutagenesis to insert new restriction sites, to alter glycosylation patterns, to change codon usage, to produce splice variants etc. Codons may be selected to increase the rate at which expression of the peptide occurs in a particular expression hosts in accordance with the frequency with which particular codons are utilised by the host (Murray E *et al*. (1989) *Nuc Acids Res* 17: 477-508). Other reasons for substantially altering the nucleotide sequence(s) of the invention and their derivatives, without altering the encoded amino acid sequences include the production of RNA transcripts having more desirable properties, such as a greater half life, than transcripts produced from naturally occurring sequences.

Additionally the invention provides a transformed plant cell or plant comprising a vector according to the invention and seed obtained from a transformed plant comprising a vector according to the invention.

The invention now provides a method of producing asexually derived embryos comprising: i) transforming a plant cell with the vector comprising a nucleotide sequence of the invention which encodes a protein which when present at a sufficient level within the plant cell renders the plant cell embryonic, or increases the regenerative capacity of the plant cell or both renders said cell embryogenic and increases the regenerative capacity of said plant cell; ii) growing said plant cell to produce tissue; iii) selecting said tissue for the presence of a nucleotide sequence of the invention; and iv) assaying said tissue for asexual embryo production; v) maintaining or growing isolated mature or immature embryos in culture.

Regeneration", as used herein, refers to a morphogenetic response that results in the production of new tissues, organs, embryos, whole plants or fragments of whole plants that are derived from a single cell, or a group of cells.

Regeneration may proceed indirectly via a callus phase or directly, without an intervening callus phase. "Regenerative capacity" refers to the ability of a plant cell to undergo regeneration.

By "embryogenic cell", it is meant a cell that has completed the transition from either a somatic or a gametophytic cell to a state where no further applied stimuli are necessary to produce a somatic or gametophytic embryo, respectively.

Also included in the invention is a method of producing asexually derived embryos comprising: i) introducing into said plant cell a protein according to the invention, which when present at a sufficient level within the plant cell renders the plant cell embryonic, or increases the regenerative capacity of the plant cell or both renders said cell embryogenic and increases the regenerative capacity of said plant cell; to produce a modified plant cell; ii) growing said modified plant cell to produce tissue; and iii) assaying said tissue for asexual embryo formation; v) maintaining or growing isolated mature or immature embryos in culture.

A further embodiment of the invention is a method for producing an apomictic plant comprising: i) transforming a plant with a vector comprising a nucleotide sequence of the invention which encodes a protein which when present at a sufficient level within the plant cell renders the plant cell embryonic, or increases the regenerative capacity of the plant cell or both renders said cell embryogenic and increases the regenerative capacity of said plant cell; to produce a transformed plant; or introducing into said plant a protein according to the invention which when present at a sufficient level within the plant cell renders the plant cell embryonic, or increases the regenerative capacity of the plant cell or both renders said cell embryogenic and increases the regenerative capacity of said plant cell; ii) selecting said transformed plant for the presence of a nucleotide sequence of the invention; and/or iii) assaying said transformed plant for asexual embryo production; iv) maintaining or growing isolated mature or immature embryos in culture; v) production of an apomictic plants from the mature embryos of step iv.

A protein of the invention can be introduced/translocated into a plant or plant cell as outlined in PCT/NL01/00388, herein encompassed by reference.

In particular said invention relates to an above method wherein said step of assaying involves assaying for asexually derived embryos, somatic embryos, gametophytically derived embryos, adventitious embryony, diplospory or for haploid parthenogenesis of the embryo sac.

The invention further provides a method of modifying the regenerative capacity of a plant comprising: i) transforming a plant cell with the vector comprising a nucleotide sequence of the invention which encodes a protein which when present at a sufficient level within the plant cell renders the plant cell embryonic, or increases the regenerative capacity of the plant cell or both renders said cell embryogenic and increases the regenerative capacity of said plant cell, or introducing into said plant cell a protein according to the invention which when present at a sufficient level within the plant cell renders the plant cell embryonic, or increases the regenerative capacity of the plant cell or both renders said cell embryogenic and increases the regenerative capacity of said plant cell; to produce a modified plant cell: ii) growing said modified plant cell to produce tissue; and iii) assaying said tissue for enhanced regeneration as compared to wild-type tissue.

Preferably the step of growing said transformed plant cell, the step of assaying said tissue, or both the step of growing said transformed plant cell and the step of assaying said tissue are carried out in the absence of an additional growth regulator.

In yet another aspect the invention provides a method of selecting a modified plant comprising; i) transforming a normally non-regenerative plant with a vector comprising a nucleotide sequence of the invention which encodes a protein which when present at a sufficient level within the plant cell renders the plant cell embryonic, or increases the regenerative capacity of the plant cell or both renders said cell embryogenic and increases the regenerative capacity of said plant cell; or introducing into said plant a protein according to the invention which when present at a sufficient level within the plant cell renders the plant cell embryonic, or increases the regenerative capacity of the plant cell or both renders said cell embryogenic and increases the regenerative capacity of said plant cell, to produce said modified plant; and ii) determining whether said modified plant is able to regenerate under conditions in which said normally non-regenerative plant does not regenerate.

A plant protein of the present invention acting as a transcriptional regulator, with it's ability to control plant growth or developmental processes, can also be used to drive the ectopic expression of a protein of interest (i.e. a protein not normally expressed in said plant), under the control of a regulatory element, wherein said regulatory element is induced by said protein. This would enable, for example, the production of a 'protein of interest' in the seeds of apomictic plants.

The invention thus provides a method of producing a protein of interest comprising; i) transforming a plant with a vector according to the invention and a second vector comprising a nucleotide sequence encoding a protein of interest under the control of a regulatory element, wherein said regulatory element is induced by the expression product of a nucleotide sequence according to the invention; to produce a transformed plant; ii) selecting said transformed plant for occurrence of a nucleotide sequence according to the invention; and iii) growing said transformed plant in order to produce said protein of interest.

As a way of illustration, a nucleotide sequence encoding a protein of interest according to the invention may be ligated to a nucleotide sequence encoding a protein or fragment thereof of the invention to form a fusion protein. The two sequences may be cleaved by inclusion of a 'cleavage' sequence (e.g. sequence (2A) of the foot and mouth disease virus and like sequences) which encodes a postranslational cleavage site between the two nucleic acid sequences. This sequence can mediate cleavage in a heterologous protein context in a range of eukaryotic expression systems.

Preferably the expression of nucleotide sequences of the invention may be driven by a number of promoters/regulatory elements, particularly active in plants, well-known in the art and readily obtainable by the skilled person. Examples of such promoters include constitutive promoters akin to viral based promoters (e.g. CaMV 35S promoter), chimeric promoters like ferredoxin/RolD and the like, tissue specific promoters like root, shoot and epidermal, endosperm specific promoters (e.g. Rubisco (shoot specific), ferredoxin (shoot specific, RolD (root specific), lipid transfer protein (LTP1), *Arabidopsis* thaliana meristem layer 1 (ATML1; epidermis specific) and Cyc promoter, B₁- or D-hordein promoter, UFO promoter (shoot meristem specific promoter) and the like. Also included are inducible and developmentally regulated promoters maize ubiquitin promoter, the cell division cycle promoter cdc2, ACT2 promoter from *Arabidopsis thaliana*, heat shock inducible promoter, pathogen inducible promoters, stress inducible promoters (like chitinase promoter) and the like. Further included are promoters that can be generated by one of skill in the art. The invention further contemplates the use of the individual promoters of the nucleotide sequences of the present invention for this purpose. At the other end of the construct a terminator is provided which causes transcription to cease. This can be any terminator which functions in plants. Particularly preferred are the NOS, OCS and 35S terminator or the potato protease inhibitor II (potpiII) terminator. When expression is mediated by the 35S promoter, particularly the cotyledons of transgenic seedlings are sites where ectopic organogenesis and somatic embryogenesis can be observed.

By a nucleotide sequence encoding a "protein of interest" it is meant by way of illustration any nucleotide that is to be expressed in a transformed plant. Such a nucleotide sequence encoding a protein of interest may include, but is not limited to, a nucleotide that encodes a pharmaceutically active protein, for example growth factors, growth regulators, antibodies, antigens, their derivatives useful for immunization or vaccination and the like. Such proteins include, but are not limited to, interleukins, insulin, G-CSF, GM-CSF, hPG-CSF, M-CSF or combinations thereof, interferons, for example, interferon-alpha , interferon- beta , interferon- tau , blood clotting factors, for example, Factor VIII, Factor IX, or tPA or combinations thereof. A nucleotide sequence of interest may also encode an industrial enzyme, protein supplement, nutraceutical, or a value-added product for feed, food, or both feed and food use. Examples of such proteins include, but are not limited to proteases, oxidases, phytases chitinases, invertases, lipases, cellulases, xylanases, enzymes involved in oil biosynthesis etc. Other protein supplements, nutraceuticals, or a value-added product etc. Preferably said protein of interest is selected from the group consisting of a pharmaceutically active protein, antibody, industrial enzyme, protein supplement, nutraceutical, storage protein, an enzyme involved in oil biosynthesis, animal feed, and animal feed supplement.

The invention further concerns a method to modulate plant growth or developmental processes in a plant or plant cell, characterised in that a nucleotide sequence of the invention or a vector comprising a nucleotide sequence of the invention is introduced into said plant or plant cell.

Modulation as used herein refers to the alteration in a positive or negative fashion of plant growth and development processes, for example through activation or suppression of gene(s) which are essentially capable of initiation, progression, suppression or repression of plant growth and development processes.

A host strain may be chosen for its ability to modulate the expression of the inserted sequences or to process the expressed protein in the desired fashion. Such modifications of the polypeptide include, but are not limited to, acylation, carboxylation, glycosylation, phosphorylation and lipidation. Post translation processing which cleaves a 'prepro' form of the protein may also be important for correct insertion, folding and/or function. Different host cells which have the correct cellular machinery and characteristic mechanisms for such post-translational activities maybe chosen to ensure correct modification and processing of the introduced, foreign protein.

In the case of the 'plant cell' officiating as 'host cell', the target 'plant cell' may be part of a whole plant or may be an isolated cell or part of a tissue which may be regenerated into a whole plant. Although the invention is illustrated in transgenic *Arabidopsis*, the actual applicability of the invention is not limited to this plant species. The target plant may be selected from any monocotyledonous or dicotyledonous plant species.

The procedure or method for preparing a transformant can be performed according to the conventional technique used in the fields of molecular biology, biotechnology and genetic engineering. Manipulation of DNA sequences in plant cells may be carried out using the Cre/loxP site specific recombination system as outlined in patent application WO9109957. An entire plant can be generated from a single transformed plant cell through culturing techniques known to those skilled in the art. Regeneration of plants from transformed material can be accomplished through somatic embryogenesis (the structures formed are bipolar with cotyledons and roots) or organogenesis (formation of shoots or roots).

Some plant species as yet remain recalcitrant in culture, not forming shoots or embryos even under a multitude of different culture conditions, the practising of the invention in such plant species is merely a matter of time and not a matter of principle, because the amenability to genetic transformation as such is of no relevance to the underlying concept of the invention.

Efficient transformation and regeneration methods are a priority for successful application of genetic engineering to the improvement of vegetative propagated plants. Transformation of plant species is now routine for an impressive number of plant species, including both the *Dicotyledoneae* as well as the *Monocotyledoneae.* In principle any transformation method may be used to introduce chimeric DNA according to the invention into a suitable ancestor cell. Methods may suitably be selected from the calcium/polyethylene glycol method for protoplasts (Krens, F.A. *et al*., 1982, Nature 296, 72-74; Negrutiu I. *et al*, June 1987, Plant Mol. Biol. 8, 363-373), electroporation of protoplasts (Shillito R.D. *et al*., 1985 Bio/Technol. 3, 1099-1102), microinjection into plant material (Crossway A. *et al*., 1986, Mol. Gen. Genet. 202, 179-185), (DNA or RNA-coated) particle bombardment of various plant material (Klein T.M. *et al*., 1987, Nature 327, 70), infection with (non-integrative) viruses, in planta *Agrobacterium tumefaciens* mediated gene transfer by infiltration of adult plants or transformation of mature pollen or microspores (EP 0 301 316) and the like. A preferred method according to the invention comprises *Agrobacterium-mediated* DNA transfer. Especially preferred is the use of the so-called binary vector technology as disclosed in EP A 120 516 and U.S. Patent 4,940,838).

Although considered somewhat more recalcitrant towards genetic transformation, monocotyledonous plants are amenable to transformation and fertile transgenic plants can be regenerated from transformed cells or embryos, or other plant material. Presently, preferred methods for transformation of monocots are microprojectile bombardment of embryos, explants or suspension cells, and direct DNA uptake or (tissue) electroporation (Shimamoto, *et al*, 1989, Nature 338, 274-276). Transgenic maize plants have been obtained by introducing the *Streptomyces hygroscopicus bar-*gene, which encodes phosphinothricin acetyltransferase (an enzyme which inactivates the herbicide phosphinothricin), into embryogenic cells of a maize suspension culture by microprojectile bombardment (Gordon-Kamm, 1990, Plant Cell, 2, 603-618). The introduction of genetic material into aleurone protoplasts of other monocot crops such as wheat and barley has been reported (Lee, 1989, Plant Mol. Biol. 13, 21-30). Wheat plants have been regenerated from embryogenic suspension culture by selecting embryogenic callus for the establishment of the embryogenic suspension cultures (Vasil, 1990 Bio/Technol. 8, 429-434). The combination with transformation systems for these crops enables the application of the present invention to monocots.

Monocotyledonous plants, including commercially important crops such as rice, wheat and corn are also amenable to DNA transfer by *Agrobacterium* strains (*vide* WO 94/00977; EP 0 159 418 B1; EP 0 856 060; Gould J, Michael D, Hasegawa O, Ulian EC, Peterson G, Smith RH, (1991) Plant. Physiol. 95, 426-434).

Generally after transformation, plant cells or cell groupings are selected for the transfer with the nucleic acid sequence encoding the protein according to the invention, following which the transformed material is regenerated into a whole plant.

Any number of selection systems may be used to recover the transformed cells (those containing recombinant DNA) over untransformed cells. Examples of suitable markers include genes that provide antibiotic or herbicide resistance. Thus anti-metabolite, antibiotic or herbicide resistance can be used as the basis of selection; for example, dhfr which confers resistance to methotrextate (Wiger M. *et al*. (1980) *Proc Natl Acad* Sci 77: 3567-70), npt, which confers resistance to the aminoglycosides neomycin and G-418 (Colbere-Garapin F. *et al*. (1981), *J Mol Biol* 150: 1-14) and *als* and *pat,* which confer resistance to chlorsulfuron and phosphinotricin acyl transferase, respectively (Murry supra). Additional selectable genes have been described, for example, trpB, which allows cells utilise indole in place of tryptophan, or His D, which allows cells to utilise histinol in place of histidine (Hartman SC and RC Mulligan (1988) *Proc Natl Acad Sci* 85: 8047-51). Alternatively one could use visible markers such as anthocyanins, beta-glucuronidase (GUS) and its substrates e.g. X-gluc, , GFP and variants, and luciferase and its substrate, luciferin, which are widely used to identify transformants, but also to quantify the amount of stable protein expression attributable to a specific vector system (Rhodes CA *et al*. (1995) *Methods Mol Biol* 55: 121-131.

Following DNA transfer and regeneration, putatively transformed plants may be evaluated, for instance using Southern analysis, for the presence of the recombinant DNA according to the invention, copy number and/or genomic organization. In addition, or alternatively, expression levels of the newly introduced DNA may be undertaken, using Northern and/or Western analysis, and other techniques well known to persons having ordinary skill in the art.

The invention further provides a host cell transformed with a nucleotide sequence of the invention. The invention further concerns a method to modulate plant growth or developmental processes, wherein said developmental comprises organogenesis and/or embryogenesis, preferably ectopic organogenesis or somatic embryogenesis, in a plant or plant cell, characterised in that a nucleotide sequence of the invention or a vector comprising a nucleotide sequence of the invention is introduced into said plant or plant cell.

It is disclosed in the examples that through transcriptional activation studies in yeast, that proteins of the invention can directly or indirectly activate transcription without being fused with transcriptional activators such as the GAL4 activation domain from yeast. Furthermore, overexpression of proteins of the invention was shown to modulate the growth, and to trigger ectopic organogenesis and somatic embryogenesis, particularly in the cotyledons of transgenic seedlings.

Also provided in the invention is a method to modulate plant growth or developmental processes in a plant or plant cell as mentioned above, further providing said plant or plant cell with a growth regulator, preferably a brassinosteroid, even more preferred 24-epibrassinolide. It has been discovered that addition of 24-epibrassinolide (C28H408, Duchefa) can mimic some of the effects of overexpression of the proteins of the invention when applied on wild-type Arabidopsis. Further the examples show that plant growth regulators, more in particular plant growth regulators belonging to the brassinosteroids, preferably 24-epibrassinolide, when used in combination with the ectopic expression of proteins of the invention, can enhance developmental processes in a plant or plant cell, more in particular ectopic organogenesis or somatic embryogenesis.

A further aspect of the invention is the use of a nucleotide sequence according to the invention or a vector according comprising a nucleotide sequence of the invention, to modulate plant growth or developmental processes in a plant or plant cell. In a preferred embodiment said developmental process comprises organogenesis and/or embryogenesis, preferably ectopic organogenesis or somatic embryogenesis. Further provided is the use of a nucleotide sequence according to the invention or a vector comprising a nucleotide sequence of the invention, in combination with a growth regulator, preferably a brassinosteroid, to modulate plant growth or developmental processes in a plant or plant cell.

A specific embodiment of the invention is a transgenic plant or plant cell obtainable by a method according to the invention. The invention now provides a plant and propagating material thereof which contains in its genome a nucleotide sequence of the invention or a vector as defined above, and which, accordingly, show a phenotype in which the development of plant cells or tissues is altered towards a more organogenic/embryogenic phenotype. Thus a genetically engineered plant or offspring of a transgenic/genetically engineered plant is encompassed by the present invention.
From the examples it will become clear that the phenotype which will show after transformation as a result of the expression of a nucleotide sequence according to the invention depends on the level of expression of the transgene (in combination with tissue and development specificity). High expression is shown to result in such severe phenotypes that no transformants could be obtained. Medium expression is most preferable for the effects on growth and development such as ectopic embryogenesis. In case of low expression the transgene can function as enhancer for regeneration as discussed herein.

Further provided is the use of material from a transgenic plant or plant cell according to the invention, to modulate plant growth or developmental processes, preferably ectopic organogenesis or somatic embryogenesis, in a non-transformed plant or plant cell. Transgenic material harbouring constructs leading to the ectopic production of a protein of the invention, can be used to initiate, growth and developmental process, more in particular embryogenesis or organogenesis, in a non-transformed plant or plant cell. Thus through the ectopic expression of one or more proteins of the invention, plant cells and tissues (including non-transformed cells or tissues) can be steered towards a more organigenic/embryogenic phenotype.

Optimisation of plant growth and development process, like somatic embryogenesis or organogenesis, is essential for the propagation of plants for commercial or conservational purposes. The ability to modulate such processes at the molecular level, more specifically through the expression of genes encoding transcriptional regulators central to these processes as provided by the present invention, paves the way for the micropropagation of commercial species deemed recalcitrant towards genetic transformation.

### Definitions

**Plant:** -refers to eukaryotic, autotrophic organisms, which are characterised by direct usage of solar energy for their primary metabolism, their permanent cell wall and in case of multicellular individuals their open unlimited growth. In case of heterotrophic plants, the organisms are in an evolutionary context essentially derived from autotrophic plants in their structure and metabolism.
**Plant cell:**- any self-propagating cell bounded by a semi permeable membrane and containing one or more plastids. Such a cell requires a cell wall if further propagation is required. 'Plant cell', as used herein, includes without limitation, seeds, suspension cultures, embryos, meristematic regions, callous tissues, protoplasts, leaves, roots, shoots, gametophytes, sporophytes, pollen and microspores.
**Dicotyledons** (and all scientific equivalents referring to the same group of plants) form one of the two divisions of the flowering plants or angiospermae in which the embryo has two or more free or fused cotyledons.
**Monocotyledons** (and all scientific equivalents referring to the same group of plants) form one of the two divisions of the flowering plants or angiospermae in which the embryo has one cotyledon.
**Construct:**- an engineered gene unit, usually involving a gene of interest (that has been fused to a promoter), a marker gene and appropriate control sequences, to be transferred into the target tissue.
**Transformant:**- A cultured plant or plant cell, that has been genetically altered through the uptake of foreign nucleic acid.
**Explant** - the excised plant part used to initiate a tissue culture.
**Embryo culture** - in vitro development or maintenance of isolated mature or immature embryos.
**Embryogenesis** - formation of an embryo either in the seed or in tissue culture; here refers to production of embryos (embryoids) in callus or cell cultures.
**Growth regulators (hormones)** - organic compounds that influence growth and multiplication, such as cytokinins and auxins.
**Morphogenesis** - (a) the process of growth and development of differentiated structures, (b) the evolution of a structure from an undifferentiated state.
**Regeneration** - in plant cultures, a morphogenetic response to a stimulus that results in the production of organs, embryos or whole plants.
**Somatic embryogenesis** - in plant culture, the process of embryo initiation and development from vegetative or non-gametic cells.
**Vegetative propagation** - reproduction of plants using a nonsexual process involving the culture of plant parts such as stem and leaf cuttings.
**Ectopic expression**: expression of a gene out of its expected time or place.

### Examples

### Example 1: N-terminal plant-specific PRGRP(A/P/L/R)GSKNK like motif.

Proteins with the sequence identifiers PRGRP(A/P/L/R)GSKNK are so far found exclusively in higher plants (R being rare compared to A, P, and L). In the dicotelydenous plant species *Arabidopsis thaliana* 15 genes (8A, 6P, 1R) can be found by data mining and in the monocotelydenous species *Oryza sativa* (rice) 11 (6P, 2A, 3L). In *Arabidopsis* the encoded proteins range from 206 to 339 amino acids.

### The accession number of the above mentioned Arabidopsis sequences is provided as a way of illustration, in table 1.

**Table 1.**

| *Arabidopsis* sequences containing PRGRPXGSKNK (X is A, P, L or R) at the N-terminus, followed by a single DUF296 domain. Numbers are derived from Genbank entries. | | |
|---|---|---|
| genomic sequence | protein | X |
| NM_115413.1 | NP_191115.1 | P |
| NM_117526.1 | NP_567432.1 | P |
| NM_111328.1 | NP_566232.1 | A |
| NM_106300.1 | NP_177776.1 | P |
| NM_130105.1 | NP_182067.1 | A |
| NM_101943.1 | NP_173514.1 | P |
| NM_117890.2 | NP_193515.1 | P |
| NM_119705.1 | NP_195265.1 | A |
| NM_117275.1 | NP_192942.1 | A |
| NM_118410.1 | NP_194012.1 | A |
| NM_115951.1 | NP_191646.1 | A |
| NM_129855.2 | NP_181822.1 | A |
| NM_129079.1 | NP_181070.1 | A |
| NM_124348.1 | NP_199781.1 | P |
| NM_101316.1 | NP_172901.1 | R |

### Example 2. N-terminal plant-specific PRGRP(A/P/L/R)GSKNK like motif, combined with a C-terminal DUF296 domain

The sequence PRGRP(A/P/L/R)GSKNK is present in the N-terminal part of plant proteins. At position 13-20 residues downstream of the lysine residue (K), is positioned a DUF296 domain, which until the present invention had unassigned function, and which stretches out for about 120-140 amino acid residues. The remaining N-terminal amino acid sequence is much less conserved. Of the 48 DUF296 domains present in the PFAM database, 36 are found in eukaryotes and exclusively in higher plants. Presently 31 of the listed DUF296 domains are found in *Arabidopsis* and 2 in rice. This probably reflects the progress in accommodating all the available data in systems like PFAM, as it can easily be found that rice proteins containing a PRGRP(A/P/L/R)GSKNK motif contain a DUF296 domain as well. Prokaryotic proteins containing a DUF296 domain are small (around 140 amino acid residues) and are composed of not much more then just the DUF296 domain.

The DUF296 domain of plant proteins can be better defined. The DUF296 domain given in the PFAM database reads:-
"frphvlevdaGeeDivekimaFarqrprgiGcvlSaiGavsnVtLrqpdssgaaaapakeygtvtleG rfEILSLsGNsflpsesgGtAppgtgglhiHvsLAgpdGqvvGglvaGpLiAageVqVvvasfvn atrkr' (amino acids given in single letter code, conserved amino acids given in capitals)". The section of sequence "'DivekimaFarqrprgiGcvlSaiG" of the DUF296 domain fits the plant proteins much better when the G prior to the cysteine residue is deleted so that the sequence identifier reads 'DivekimaFarqrprgi**Cv**lSaiG'. In a subgroup of plant proteins containing the PRGRP(A/P/L/R)GSKNK like motif as mentioned in example 1, a serine (s) residue is found at the position of this particular conserved cysteine residue within the DUF296 domain.

### Example 3: Plant proteins containing a single N-terminal PRGRP(A/P/L/R)GSKNK like motif are involved in transcriptional regulation

A2220 bp XbaI-EcoRI promoter fragment (about -3570 to -350 upstream of the ATG translational start site) of an *Arabidopsis* gene encoding an auxin-inducible plasma membrane protein (AF098631) was fused upstream of a yeast HIS3 reporter gene in pINT1 (AF289993) after digestion of this vector with SpeI and EcoRI. After homologous recombination in yeast strain Y187 (see P.B.F. Ouwerkerk and A.H. Meijer 2001, Current Protocols in Molecular Biology 12.12.1-12.12.22, John Wiley & Sons, Inc.) it was found that no 3-AT was required to suppress growth of the recombinant yeast strain on histidine-lacking medium (28 °C). In order to find cDNA sequences encoding proteins that (putatively) interact with the *Arabidopsis* promoter sequence, a one-hybrid screening was performed in yeast. The cDNA library in lamdaACTII (see same ref) was prepared starting from a 1/1 mixture of polyA+ RNA of auxin-treated and non-treated *Arabidopsis* roots (about 7.10⁶ primary recombinant phages were obtained). After conversion to a plasmid library, the promoter-containing yeast strain was transformed with plasmid material (about 3.75.10⁶ yeast colonies obtained) and histidine-independent transformants were selected up till 6 days after transformation (at 28 °C). Most of the transformants harboured a plasmid in which the *Arabidopsis* cDNA sequence followed the yeast GAL4 activation domain (GAL4-AD) in such a manner that a translational fusion was created. However, two related cDNA sequences were recovered that were not translationally fused with the GAL4-AD. This fact indicates that the proteins encoded by these particular cDNA sequences trans-activated the HIS3 reporter gene due to direct or indirect interaction with the upstream *Arabidopsis* promoter sequence. Moreover, the proteins did not need an N-terminal fusion with yeast GAL4-AD, suggesting their expression can lead to DNA-binding as well as transcriptional activation of genes nearby the site where DNA-binding occurs. Sequence analysis demonstrated that both clones contained a complete coding sequence, corresponding to NP_191115.1 and NP_177776.1, respectively. Searching databases revealed that the *Arabidopsis* genome contains 15 genes with similar sequence characteristics (including the two mentioned). See table 1.

**Example 3:-** Overexpression of cDNA sequences encoding plant proteins containing a single PRGRP(A/P/L/R)GSKNK like motif and a single DUF296-like motif can lead to strongly altered seedling development and somatic embryogenesis
The cDNAs corresponding to NM_115413.1 and NM_106300.1 were cloned as SmaI/BglII fragments (partial digests) in SmaI/BamHI digested pART7 (A.P. Gleave, 1992, Plant Mol. Biol. 20, 1203-1207) under control of the CaMV 35S promoter. In the final wide host range vector pART27, the orientation of the NotI fragments was chosen in such a manner that the transcription as driven by the 35S promoter proceeded towards the right border (RB) sequence of the T-DNA. After floral dip transformation of *Arabidopsis* plants, it was found that overexpression of both cDNAs caused a dominant, heritable phenotype. Many of the primary transformants (T1) that could be isolated, especially those harbouring the NM_115413.1 cDNA overexpression construct, had aberrant leaf growth and were late flowering. The next generation (T2) showed severe phenotypes at the seedling stage, segregating in a mendelian fashion and linked with resistance to kanamycin due to the selectable *nptII* marker gene which was present in the transferred T-DNA. The NM_115413.1 overexpression construct often caused a nearly seedling lethal phenotype throughout T2 families with distorted, pale cotyledons and rather inactive apical meristems. In a substantial fraction of the T2 families embryonic structures were formed particularly on the cotyledons which could grow out to viable somatic embryos (see figure 1). The phenotype caused by the NM_106300.1 cDNA overexpression construct was less severe and predominantly affected cotyledon and leaf shape, being more oval or lancet shaped than wild type. In some cases callus formation was observed on older cotyledons (see figure 2).

Despite the severity of phenotypes, a fraction of the seedlings recovered and finally formed rather normal and fertile plants. In the next generation (T3) homozygous transgenic lines were selected which were propagated further (T4, T5) and also used for crossings. These experiments further demonstrated that the phenotype was stably inherited as a dominant trait, with homozygous seedlings exhibiting a (slightly) more severe phenotype than hemizygous seedlings. The ability of one of these proteins (NP_191115.1) to induce somatic embryos is clearly shown (see figure 1).

Constructs with the cDNAs corresponding to NM_115413.1 and NM_106300.1 in the reverse (anti-sense) orientation under control of the CaMV 35S promoter did not lead to transgenic plants which showed a phenotype that could be visually distinguished from wild type.

**Example 4:** Variability in effects of expression of cDNA sequences encoding plant proteins containing a single N-terminal PRGRP(A/P/L/R)GSKNK like motif and a single DUF296-like motif.

An *Arabidopsis* root transformation protocol showed that much less transformed callus/shoots were obtained when the *Agrobacterium tumefaciens* used for co-cultivation contained the NM_115413.1 overexpression construct then when the cDNA insert was not present in the construct. Seeds of the transgenic plants obtained, did not exhibit a clear phenotype. This seems to indicate that untimely expression of CAB75914.1 during this protocol is toxic for the plant cell and that only weak expressors or plants with a defective overexpression construct can be obtained. With ovule transformation as it occurs with the vacuum infiltration or flower dip method, a decreased transformation efficiency with either the NM_115413.1 or the NM_106300.1 overexpresson construct was not observed. Thus although it is possible that with this method transgenic seedlings with a very severe phenotype were not distinguishable among the seedlings that were killed by the antibioticum selection, this fraction of the total transformants does not seem to be a large one. Probably, the discrepancy between the two transformation methods is the result of the difference in activity of the 35S promoter in the initially transformed cells. While the 35S promoter is virtually inactive in ovules and embryos up to the globular stage, it is usually very active in callus cells. It thus might be that those callus-like cells transformed with the root transformation protocol do not further develop due to immediate toxicity of NP_191115.1 when present at high levels in these cells (possibly also due to the combination of exogenous addition of plant hormones), while during zygotic embryogenesis the initial transgenic cells have a better chance to develop into multicellular structures.

### Example 5: Influence of plant hormones

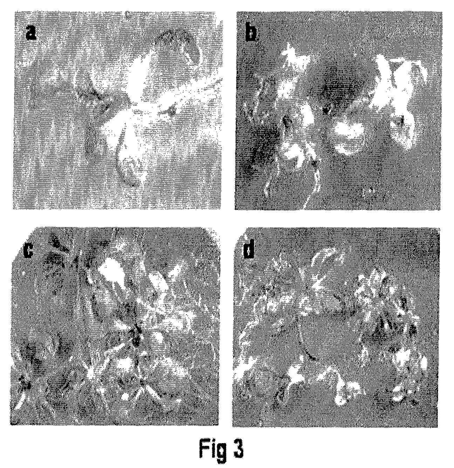

Of the plant growth substances investigated, 24-epibrassinolide had the most profound effect on wild type plants when one considers the ability to mimic the mutant phenotypes caused by NM_115413.1/NM_106300.1 overexpression constructs although somatic embryogenesis was not observed (see figure 3). 24-epibrassinolide was also able to make the mutant phenotypes more vigorous.

This suggested that overexpression of proteins with the sequence identifiers mentioned above results in altered brassinolide levels in plant cells. It is thus likely that overexpression directly or indirectly leads to changes in the levels of plant growth substances. Plant cells affected in this manner can secrete regulatory signals affecting neighbouring cells or tissues. It is thus shown herein that by using plant material overexpressing one or more members of the protein family described with the sequence identifiers mentioned, the development of other plant material (including non-transformed material) can be altered towards a more organogenic/embryogenic pathway.

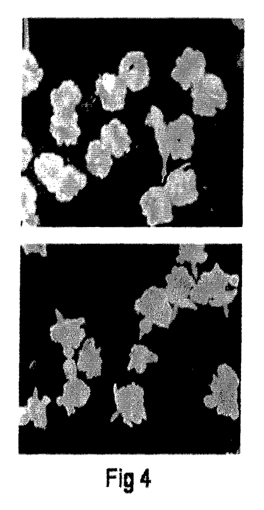

When seeds of NM_115413.1 overexpressing plants were germinated and grown in liquid 2,4-dichlorophenoxy-acetic acid (2,4-D) containing medium (as described by A.P. Mordhorst et al., 1998, Genetics 149, 549-563), it was noted that they developed into structures with relatively large meristematic apical zones. Compared to control lines, unorganized callus growth on cotyledons was very much restricted (see figure 4).

## Claims

1. A plant protein involved in transcriptional regulation **characterised in that** it comprises the amino acid sequence PRGRPXGSKNK, wherein X is A, P, L or R.

2. A plant protein according to claim 1, **characterised in that** it also comprises a DUF296 domain.

3. A plant protein according to claim 2, **characterised in that** it has at least 200 amino acids, whereby the amino acid sequence PRGRPXGSKNK is present in the N-terminal part, and wherein the DUF296 domain is separated from said sequence by about 13-20 amino acid residues.

4. A plant protein according to claim 3, **characterised in that** a cysteine to serine substitution has taken place in the DUF296 domain.

5. A plant protein according to any of claims 1-4, **characterised in that** it comprises the amino acid sequence to NP_191115.1 or NP_177776.1, or a functional part thereof.

6. A nucleotide sequence encoding a protein according to any of claims 1-5.

7. A nucleotide sequence according to claim 6, **characterised in that** it comprises a nucleotide sequence coding for NP_177776.1 or coding for NP_191115..

8. A nucleotide sequence according to claim 7, **characterised in that** it comprise the nucleotide sequence NM_115413.1 or NM_106300.1.

9. Vector comprising a nucleotide sequence according to any of claims 6 to 8.

10. A transformed plant cell comprising a vector according to claim 9.

11. A transformed plant comprising a vector according to claim 9.

12. A seed obtained from a transformed plant of claim 11.

13. A method to modulate plant growth or developmental processes in a plant or plant cell, **characterised in that** a protein according to claims 1-5, or a nucleotide sequence according to claims 6-8 or a vector according to claim 9 is introduced into said plant or plant cell.

14. A method according to claim 13, **characterised in that** said developmental process comprises organogenesis and/or embryogenesis, preferably ectopic organogenesis or somatic embryogenesis.

15. A method according to claim 13 or claim 14, further providing said plant or plant cell with a growth regulator, preferably a brassinosteroid.

16. A method for producing asexually derived embryos comprising:
i) transforming a plant cell with the vector according to claim 9;
ii) growing said plant cell to produce tissue;
iii) selecting said tissue for the presence of a nucleotide sequence according to claims 6-8; and
iv) assaying said tissue for asexual embryo production;
v) maintaining or growing isolated mature or immature embryos in culture.

17. A method for producing asexually derived embryos comprising:
i) introducing into said plant cell a protein according to any of claims 1-5, to produce a modified plant cell;
ii) growing said modified plant cell to produce tissue; and
iii) assaying said tissue for asexual embryo formation;
iv) maintaining or growing isolated mature or immature embryos in culture.

18. A method for producing an apomictic plant comprising:
i) transforming a plant with the vector according to claim 9, to produce a transformed plant; or introducing into said plant a protein according to any of claims 1-5;
ii) selecting said transformed plant for the presence of a nucleotide sequence according to claims 6-8; and/or
iii) assaying said transformed plant for asexual embryo production;
iv) maintaining or growing isolated mature or immature embryos in culture;
v) production of an apomictic plants from the mature embryos of step iv.

19. The method of claim 16-18, wherein the step of assaying involves assaying for adventitious embryony.

20. The method of claim 16-18, wherein the step of assaying involves assaying for somatic embryos.

21. The method of claim 16-18, wherein the step of assaying involves assaying for gametophytic embryos.

22. The method of claim 16-18, wherein the step of assaying involves assaying for parthenogenesis of the embryo sac.

23. The method of claim 16-18, wherein the step of assaying involves assaying for diplospory.

24. A method for enhancing the regenerative capacity of a plant comprising
i) transforming a plant cell with the vector according to claim 9, or introducing into said plant cell a protein according to any of claims 1-5; to produce a modified plant cell:
ii) growing said modified plant cell to produce tissue; and
iii) assaying said tissue for enhanced regeneration as compared to wild-type tissue;
iv) regeneration of complete plants from said tissue.

25. The method of claim 19, wherein the step of growing said transformed plant cell, the step of assaying said tissue, or both the step of growing said transformed plant cell and the step of assaying said tissue are carried out in the absence of an additional growth regulator.

26. A method of selecting a modified plant comprising;
i) transforming a normally non-regenerative plant with a vector according to claim 9; or introducing into said plant a protein according to any of claims 1-5, to produce said modified plant; and
ii) determining whether said modified plant is able to regenerate under conditions in which said normally non-regenerative plant does not regenerate.

27. A method of producing a protein of interest comprising;
i) transforming a plant with a vector according to claim 9 and a second vector comprising a nucleotide sequence encoding a protein of interest under the control of a regulatory element, wherein said regulatory element is induced by the expression product of a nucleotide sequence according to claims 6-8; to produce a transformed plant;
ii) selecting said transformed plant for occurrence of a nucleotide sequence according to claims 6-8; and
iv) growing said transformed plant in order to produce said protein of interest.

28. The method of claim 27, wherein said protein of interest is selected from the group consisting of a pharmaceutically active protein, antibody, industrial enzyme, protein supplement, nutraceutical, storage protein, an enzyme involved in oil biosynthesis, animal feed, and animal feed supplement.

29. Use of a protein according to claims 1-5, or nucleotide sequence according to claims 6-8 or a vector according to claim 9, to modulate plant growth or developmental processes in a plant or plant cell.

30. Use according to claim 28, wherein said developmental process comprises organogenesis and/or embryogenesis, preferably ectopic organogenesis or somatic embryogenesis.

31. Use according to claim 29 or claim 30 in combination with a growth regulator, preferably a brassinosteroid.

32. A transgenic plant or plant cell obtainable by a method according to any of claims 13-28.

33. Use of material from a transgenic plant or plant cell according to claim 32, to modulate plant growth or developmental processes, preferably ectopic organogenesis or somatic embryogenesis, in a non-transformed plant or plant cell.
